# EUROPÄISCHE PATENTSCHRIFT

(11) **EP 0 602 523 B1**
(45) Veröffentlichungstag und Bekanntmachung des Hinweises auf die Patenterteilung: **11.12.1996**
(21) Anmeldenummer: 93119782.6
(22) Anmeldetag: 08.12.1993
(51) Int. Cl.: C07D 213/70, A61K 31/44, C07D 213/65, C07D 213/64, C07D 217/24, C07D 215/20, C07D 215/26, C07D 207/32, A61K 31/47, C07D 521/00

(54) **Substituierte Benzoylguanidine, Verfahren zu ihrer Herstellung, ihre Verwendung als Medikament oder Diagnostikum sowie sie enthaltendes Medikament**
Substituted benzoylguanidines, process for their preparation, their use as medicament or diagnostic agent, as well as a medicament containing them
Benzoylguanidines substituées, procédé pour leur préparation leur utilisation comme médicament ou agent diagnostique, ainsi que médicament les contenant

(30) Priorität: 15.12.1992 DE 4242191; 09.04.1993 DE 4311800
(43) Veröffentlichungstag der Anmeldung: 22.06.1994
(73) Patentinhaber: HOECHST AKTIENGESELLSCHAFT, 65929 Frankfurt am Main (DE)
(72) Erfinder: Lang, Hans-Jochen, Dr., D-65719 Hofheim/Taunus (DE); Kleemann, Heinz-Werner, Dr., D-61350 Bad Homburg (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 672
- BIOCHEMICAL AND BIOPHYSICAL RESEARCH COMMUNICATIONS Bd. 184, Nr. 1 , 15. April 1992 Seiten 112 - 117 A. SCHMID ET AL.

## Beschreibung

Die Erfindung betrifft Benzoylguanidine der Formel I 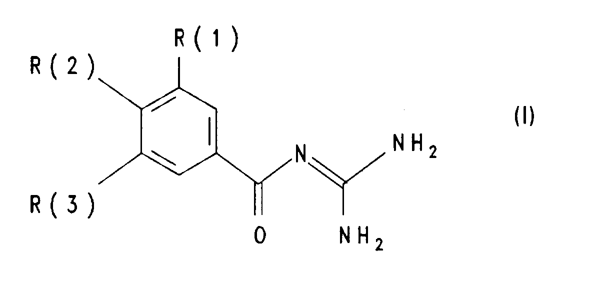 worin bedeuten:
- R(1): Wasserstoff, F, Cl, Br, I, -NO₂, -C ≡ N, R(16)-CₚH₂ₚ-O_{q}, R(4)-SOₘ oder R(5)R(6)N-SO₂-, mit
- m: Null, 1 oder 2;
- p: Null oder 1;
- q: Null oder 1;
- R(16): CᵣF₂ᵣ₊₁;
- r: 1, 2 oder 3;
- R(4) und R(5): (C₁-C₈)-Alkyl, (C₃-C₆)-Alkenyl, -CₙH₂ₙ-R(7) oder CF₃;
- n: Null, 1, 2, 3 oder 4;
- R(7): (C₃-C₇)-Cycloalkyl oder Phenyl,
welches nicht substituiert ist oder substituiert mit 1 - 3 Substituenten aus der Gruppe F, Cl, CF₃, Methyl, Methoxy oder NR(8)R(9);
R(8) und R(9)
H oder C₁-C₄-Alkyl;
oder
R(5) auch H;
R(6) H oder (C₁-C₄)-Alkyl;
oder
- R(5) und R(6): gemeinsam 4 oder 5 Methylengruppen, von denen eine CH₂-Gruppe durch Sauerstoff, S, NH, N-CH₃ oder N-Benzyl ersetzt sein kann;
- R(2): (C₁-C₉)-Stickstoff-Heteroaryl,
das über C oder N verknüpft ist und das unsubstituiert oder substituiert ist mit 1 - 3 Substituenten aus der Gruppe F, Cl, CF₃, CH₃, Methoxy, Hydroxy, Amino, Methylamino oder Dimethylamino;
oder
- R(2): -SR(10), -OR(10) oder -NR(10)R(11);
- R(10): -CₐH₂ₐ-(C₁-C₉)-Stickstoff-Heteroaryl,
das unsubstituiert oder substituiert ist mit 1 - 3 Substituenten aus der Gruppe F, Cl, CF₃, CH₃, Methoxy, Hydroxy, Amino, Methylamino, Dimethylamino;
- a: Null, 1 oder 2;
- R(11) und R(12): unabhängig voneinander wie R(10) definiert oder Wasserstoff oder (C₁-C₄)-Alkyl;
- R(3): wie R(1) definiert, oder (C₁-C₆)-Alkyl, -X-R(13);
- X: Sauerstoff, S oder NR(14);
R(14) H oder (C₁-C₃)-Alkyl;
- R(13): H, (C₁-C₆)-Alkyl, (C₃-C₈)-Cycloalkyl oder -C_{b}H_{2b}-R(15);
- b: Null, 1, 2, 3 oder 4;
oder
- R(13) und R(14): gemeinsam 4 oder 5 Methylengruppen, von denen eine CH₂-Gruppe durch Sauerstoff, S, NH, N-CH₃ oder N-Benzyl ersetzt sein kann;
- R(15): Phenyl,
das unsubstituiert oder substituiert ist mit 1 - 3 Substituenten aus der Gruppe F, Cl, CF₃, Methyl, Methoxy oder NR(8)R(9);
R(8) und R(9)
H oder (C₁-C₄)-Alkyl;
sowie deren pharmazeutisch verträgliche Salze;
mit Ausnahme von Verbindungen I, in denen gleichzeitig bedeuten:
- R(1): R(4)-SOₘ oder R(5)R(6)NSO₂, und
- R(2): (C₁-C₉)-Heteroaryl,
sowie mit Ausnahme der Verbindung 4-(3-Pyridyloxy)-3-N-pyrrolidinobenzoesäure-guanidin.

Bevorzugt sind Verbindungen der Formel I, in der bedeuten:
- R(1): Wasserstoff, F, Cl, -C ≡ N, R(16)-CₚH₂ₚ-O_{q}, R(4)-SOₘ oder R(5)R(6)N-SO₂-;
- p: Null oder 1;
- q: Null oder 1;
- R(16): CᵣF₂ᵣ₊₁;
- r: 1, 2 oder 3;
- m: Null, 1 oder 2;
- R(4) und R(5): (C₁-C₈)-Alkyl, (C₃-C₄)-Alkenyl, -CₙH₂ₙ-R(7) oder -CF₃;
- n: Null oder 1;
- R(7): (C₃-C₆)-Cycloalkyl oder Phenyl,
welches unsubstituiert ist oder substituiert mit 1-3 Substituenten aus der Gruppe F, Cl, CF₃, Methyl, Methoxy oder NR(8)R(9);
R(8) und R(9)
H oder Methyl;
oder
- R(5): H;
R(6) H oder Methyl;
- R(3): Wasserstoff, Methyl, Cyano, -CF₃, F oder Cl;
und die übrigen Reste wie oben definiert sind,
sowie deren pharmazeutisch verträgliche Salze;
mit Ausnahme von Verbindungen I, in denen gleichzeitig bedeuten:
- R(1): R(4)-SOₘ oder R(5)R(6)NSO₂, und
- R(2): (C₁-C₉)-Heteroaryl,
und mit Ausnahme der Verbindung 4-(3-Pyridyloxy)-3-N-pyrrolidinobenzoesäureguanidin.

Besonders bevorzugt sind Verbindungen I, in denen bedeuten:
- R(1): Wasserstoff, F, Cl, -C ≡ N, -CF₃, R(4)-SOₘ oder R(5)R(6)N-SO₂-;
- m: Null, 1 oder 2;
- R(4): Methyl oder CF₃;
oder
- R(5) und R(6): unabhängig voneinander H oder Methyl;
- R(2): (C₁-C₉)-Stickstoff-Heteroaryl,
das über C oder N verknüpft ist und das unsubstituiert oder substituiert ist mit einem Rest aus der Reihe F, Cl, CF₃, CH₃, Methoxy und Dimethylamino;
oder
- R(2): -SR(10), -OR(10) oder -NR(10)R(11);
- R(10): -CₐH₂ₐ-(C₁-C₉)-Stickstoff-Heteroaryl, das unsubstituiert oder substituiert ist mit einem Rest aus der Reihe F, Cl, CF₃, CH₃, Methoxy und Dimethylamino;
- a: Null, 1 oder 2;
- R(11) und R(12): unabhängig voneinander Wasserstoff oder Methyl;
- R(3): Methyl, Cyano, Trifluormethyl, F, Cl oder Wasserstoff;
sowie deren pharmazeutisch verträgliche Salze; mit Ausnahme von Verbindungen I, in denen gleichzeitig bedeuten:
- R(1): R(4)-SOₘ oder R(5)R(6)NSO₂, und
- R(2): (C₁-C₉)-Stickstoff-Heteroaryl,
und mit Ausnahme der Verbindung 4-(3-Pyridyloxy)-3-N-pyrrolidinobenzoesäureguanidin.

Unter (C₁-C₉)-Stickstoff-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind und welche wenigstens ein Stickstoff-Atom enthalten. Des weiteren können auch ein oder beide Atome der Kondensationsstelle bicyclischer Reste (wie im Indolizinyl) N-Atome sein.

Als Stickstoff-Heteroaryl gelten insbesondere, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(22) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindung I, dadurch gekennzeichnet, daß man Verbindungen der Formel II 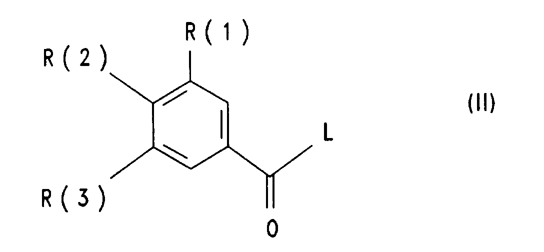 mit Guanidin umsetzt, worin R(1) bis R(3) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyl uronium-tetrafluorborat ("TOTU")[Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und III verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man beispielsweise 4-(bzw. 5-)Halogen-3-chlor-sulfonylbenzoesäuren mit Ammoniak oder Aminen in 3-Aminosulfonyl-4-(bzw. 5-)halogen-benzoesäuren bzw. mit einem schwachen Reduktionsmittel wie Natriumbisulfit und anschließender Alkylierung in 3-Alkylsulfonyl-4-(bzw. 5-)halogen-benzoesäuren überführt und die erhaltenen Benzoesäuren nach einer der oben beschriebenen Verfahrensvarianten zu erfindungsgemäßen Verbindungen I umgesetzt werden.

Die Einführung einiger Substituenten in 4- und 5-Stellung gelingt durch literaturbekannte Methoden des Palladium-vermittelten cross-couplings von Arylhalogeniden mit beispielsweise Organostannanen, Organoboronsäuren oder Organoboranen oder Organokupfer- bzw. zink-verbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 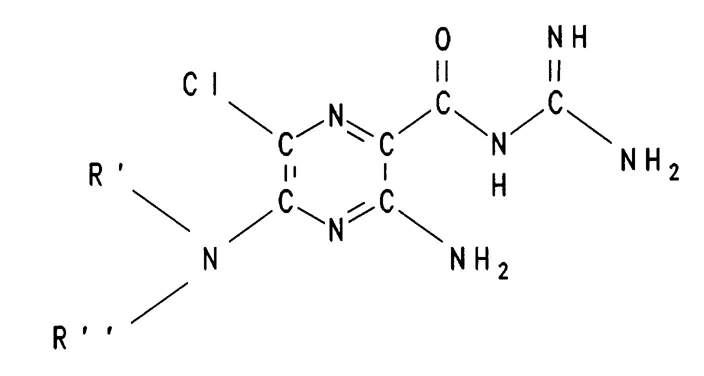 Amilorid: R',R'' = H
Dimethylamilorid: R',R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden 3,5-substituierte Benzoylguanidine vorgeschlagen, in welchen aber die Substituenten R(2) und R(3) nicht die nach der vorliegenden Erfindung beanspruchten Bedeutungen haben.

In der EP-A-556 672, die nicht vorveröffentlicht ist, wird in Beispiel 13 bereits die Verbindung 4-(3-Pyridyloxy)-3-N-pyrrolidinobenzoesäure-guanidin vorgeschlagen; sie ist von den vorliegenden Ansprüchen ausdrücklich ausgenommen.

Die EP-A-416 499 beschreibt schon ähnliche Verbindungen, jedoch erfüllen diese noch nicht alle Anforderungen; insbesondere läßt deren Wasserlöslichkeit noch zu wünschen übrig.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- NBS: N-Bromsuccinimid
- AIBN: α,α-Azo-bis-isobutyronitril
- EI: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent

### Experimenteller Teil

### Allgemeine Vorschrift zur Herstellung von Benzoyl-guanidinen (I) Variante A: aus Benzoesäuren (II, L=OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1: 4-(4-Pyridylthio)-3-methylsulfonyl-benzoylguanidin

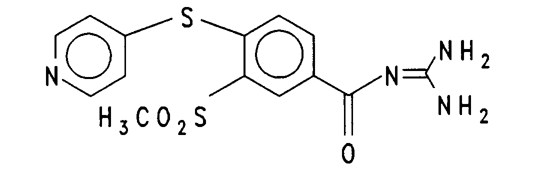

### a) 4-(4-Pyridylthio)-3-methylsulfonyl-benzoesäuremethylester

6 mmol 4-Chlor-3-methylsulfonyl-benzoesäuremethylester, 18 mmol K₂CO₃ sowie 6 mmol 4-Pyridylthiol werden in 30 ml DMF (wasserfrei) 1 h bei 130° C gerührt. Das Gemisch wird anschließend in 100 ml gesättigter wäßriger NaHCO₃-Lösung gegossen und 3 x mit 100 ml EE extrahiert. Über Na₂SO₄ wird getrocknet, das Lösungsmittel im Vakuum entfernt und an Kieselgel mit MTB chromatographiert.
Blaßgelbe Kristalle, mp 112° C,
R_{f}(MTB) = 0,17 MS (DCI) : 324 (M + 1)

### b) 4-(4-Pyridylthio)-3-methylsulfonyl-benzoylguanidin

3,6 mmol Ester 1 a) und 18,1 mmol Guanidin werden nach der allgemeinen Vorschrift B umgesetzt.
Weiße Kristalle, mp 205° C
R_{f} (EE/MeOH 5:1) = 0,24 MS (DCI) : 351 (M + 1)

Die Titelverbindung des Beispiels 2 wird analog Beispiel 1 synthetisiert:

### Beispiel 2: 4-(2-Pyridylthio)-3-methylsulfonyl-benzoylguanidin

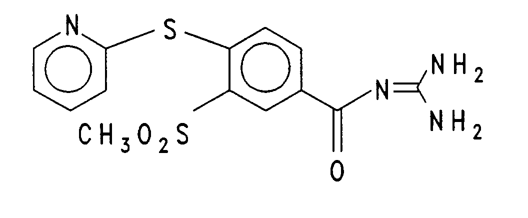

mp 207°C
R_{f} (EE/MeOH 5:1) = 0,27 MS(DCI): 351 (M + 1)

### Beispiel 3: 4-(3-Pyridyloxy)-3-methylsulfonyl-benzoylguanidin

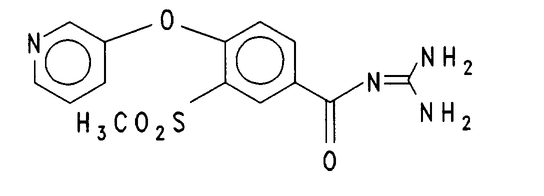

### a) 4-(3-Pyridyloxy)-3-methylsulfonyl-benzoesäuremethylester

2 mmol 4-Chlor-3-methylsulfonyl-benzoesäuremethylester, 2 mmol 3-Pyridinol sowie 6 mmol K₂CO₃ wurden in 20 ml DMF (wasserfrei) bei 130°C 2 h gerührt. Das Gemisch wird anschließend in 100 ml gesättigte wäßrige NaHCO₃-Lösung gegossen und 3 x mit 100 ml EE extrahiert. Über Na₂SO₄ wird getrocknet, das Lösungsmittel im Vakuum entfernt und das Produkt ohne weitere Reinigung weiter umgesetzt.
R_{f}(MTB) = 0,15 MS (DCI) : 308 (M + 1)

### b) 4-(3-Pyridyloxy)-3-methylsulfonyl-benzoesäure

2 mmol Ester 3 a) werden in 20 ml MeOH gelöst und mit 5 Äquivalenten 2 N wäßriger NaOH versetzt. 3 h wird bei RT gerührt, 50 ml 0,3 M wäßrige KH₂PO₄-Lösung zugegeben und 3 x mit 50 ml EE extrahiert. Über Na₂SO₄-Lösung wird getrocknet, das Lösungsmittel im Vakuum entfernt und das Produkt ohne weitere Reinigung weiter umgesetzt.
R_{f}(EE/MeOH 5:1) = 0,14 MS (DCI) : 294 (M + 1)

### c) 4-(3-Pyridyloxy)-3-methoxysulfonyl-benzoylguanidin

2 mmol Benzoesäure 3 b), 2,2 mmol Carbonyldiimidazol und 10 mmol Guanidin werden nach der allgemeinen Vorschrift A umgesetzt.
Weiße Kristalle mp 202° C,
R_{f}(EE/MeOH 5:1) = 0,38 MS (DCI) : 335 (M + 1)

Die Titelverbindungen der Beispiele 4 bis 10 werden analog Beispiel 3 synthetisiert:

### Beispiel 4: 4-(2-Pyridyloxy)-3-methylsulfonyl-benzoylguanidin

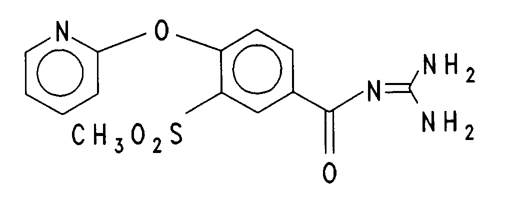

amorph;
R_{f} (EE/MeOH 5:1) = 0,41 MS (DCI): 335 (M + 1)

### Beispiel 5: 4-(4-Pyridyloxy)-3-methylsulfonyl-benzoylguanidin

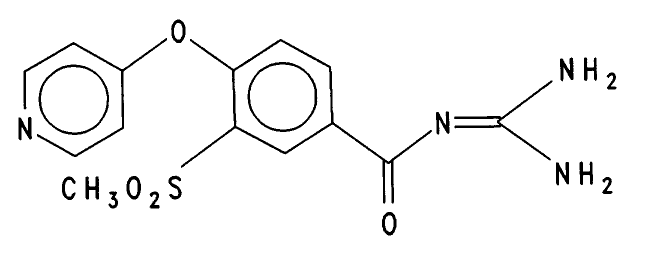

amorph;
R_{f} (EE/MeOH 3:1) = 0,22 MS (DCI): 335 (M + 1)

### Beispiel 6: 4-(7-Isochinolinoxy)-3-methylsulfonyl-benzoylguanidin

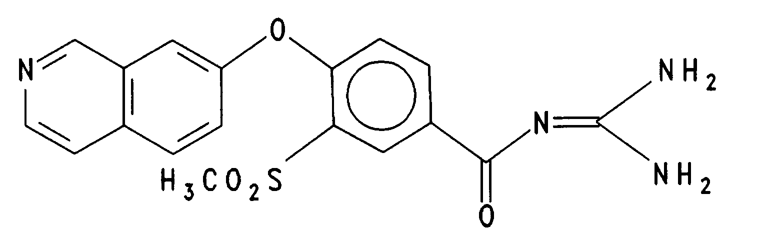

m.p. 103 bis 105°C
R_{f} (EE/MeOH 5:1) = 0,23 MS (DCI): 385 (M + 1)

### Beispiel 7: 4-(5-Isochinolinoxy)-3-methylsulfonyl-benzoylguanidin

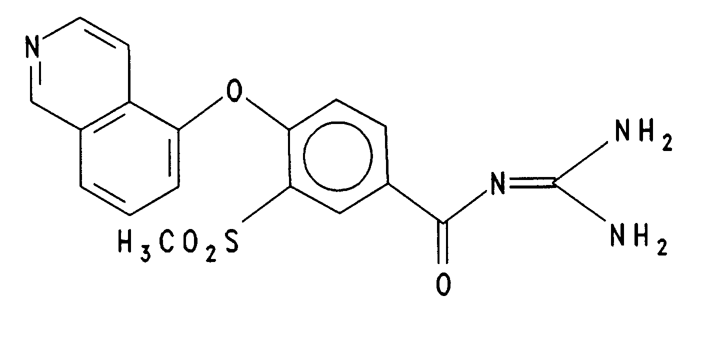

amorph;
R_{f} (EE/Toluol/MeOH 3:3:1) = 0,21 MS (DCI): 335 (M + 1)

### Beispiel 8: 4-(5-Chinolinoxy)-3-methylsulfonyl-benzoylguanidin

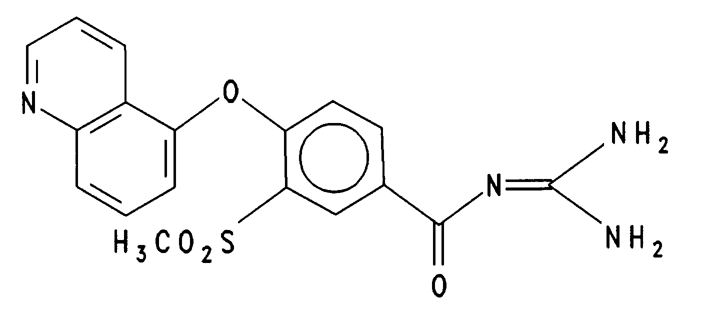

amorph;
R_{f} (EE/MeOH 5:1) = 0,23 MS (DCI): 385 (M + 1)

### Beispiel 9: 4-(6-Chinolinoxy)-3-methylsulfonyl-benzoylguanidin

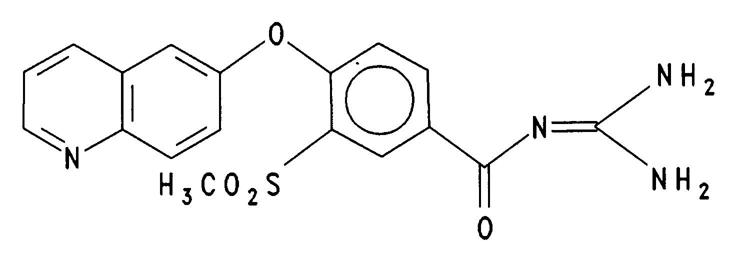

amorph;
R_{f} (EE/Toluol/MeOH 3:3:1) = 0,24 MS (DCI): 385 (M + 1)

### Beispiel 10: 4-(8-Chinolinoxy)-3-methylsulfonyl-benzoylguanidin

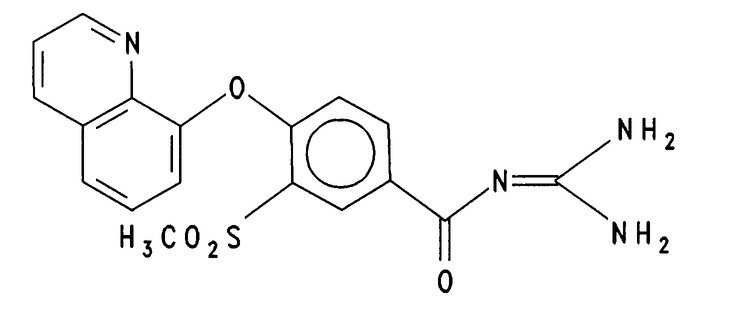

amorph;
R_{f} (EE/MeOH 5:1) = 0,23 MS (DCI): 385 (M + 1)

### Allgemeine Vorschrift zur Herstellung von 4-(1-Pyrrolo)-benzoesäuren

0.01 Mol einer 4-Aminobenzoesäure werden in 20 ml Eisessig gelöst und anschließend 0.01 Mol 2,5-Dimethoxy-tetrahydrofuran zugegeben. Man rührt 15 Minuten bei Raumtemperatur und 1 Stunde unter Siedebedingungen, läßt abkühlen und gießt das Reaktionsgemisch anschließend in 200 ml Wasser. Man filtriert den kristallinen Niederschlag ab, wäscht mehrfach mit Wasser nach und trocknet die so erhaltene 4-(1-Pyrrolo)-benzoesäure an der Luft.

Die nachfolgende Umsetzung einer 4-(1-Pyrrolo)-benzoesäure mit Guanidin nach vorhergehender Aktivierung mit Carbonyldiimidazol liefert gemäß der Allgemeinen Vorschrift A zur Herstellung von Benzoylguanidinen die entsprechenden 4-(1-Pyrrolo)benzoyl-guanidine bzw. deren Salze:

### Beispiel 11: 3,5-Dichlor-4-(1-pyrrolo)benzoyl-guanidin-hydrochlorid

erhält man gemäß Allgemeiner Vorschrift A aus 3,5-Dichlor-4-(1-pyrrolo)benzoesäure, farblose Kristalle, Schmp. 274°C.

Die 3,5-Dichlor-4-(1-pyrrolo)benzoesäure (Fp. 178 - 181°C) erhält man aus 3,5-Dichlor-4-aminobenzoesäure analog der allgemeinen Vorschrift zur Herstellung von 4-(1-Pyrrolo)-benzoesäuren

### Beispiel 12: 3-Chlor-5-methyl-4-(1-pyrrolo)benzoyl-guanidin-hydrochlorid

erhält man gemäß Allgemeiner Vorschrift A aus 3-Chlor-5-methyl-4-(1-pyrrolo)benzoesäure, farblose Kristalle, Schmp. 236 - 238°C.

Die 3-Chlor-5-methyl-4-(1-pyrrolo)benzoesäure (Fp. 160 - 162°C, Zersetzung)erhält man aus 3-Chlor-5-methyl-4-aminobenzoesäure analog der allgemeinen Vorschrift zur Herstellung von 4-(1-Pyrrolo)-benzoesäuren.

### Beispiel 13: 3,5-Dimethyl-4-(1-pyrrolo)benzoyl-guanidin-hydrochlorid

erhält man gemäß Allgemeiner Vorschrift A aus 3,5-Dimethyl-4-(1-pyrrolo)benzoesäure, farblose Kristalle, Schmp. 261 - 263°C.

Die 3,5-Dimethyl-4-(1-pyrrolo)benzoesäure (Fp. 197 - 200°C) erhält aus 3,5-Dimethyl-4-aminobenzoesäure analog der allgemeinen Vorschrift zur Herstellung von 4-(1-Pyrrolo)-benzoesäuren.

### Allgemeine Vorschrift zur Herstellung von 4-[4-(1,2,4-Triazolyl)]benzoesäuren

0.01 Mol eines 4-Aminobenzoesäuremethylesters werden unter Rührung in 40 ml siedendes Phosphoroxichlorid (POCl₃) suspendiert, nachfolgend 0.044 Mol N,N'-Diformylhydrazin zugegeben und sodann 45 Minuten am aufgesetzten Rückflußkühler gekocht. Man destilliert das überschüssige POCl₃ ab und behandelt den zumeist dunklen, viskosen Rückstand mit 200 ml einer gesättigten, wäßrigen Natriumacetat-Lösung. Der Niederschlag wird abfiltriert und mehrmals mit Wasser gewaschen.

0.01 Mol des so erhaltenen 4-[4-(1,2,4-Triazolyl)]benzoesäure-methylesters hydrolisiert man durch Kochen in einer Mischung aus 18 ml reiner Essigsäure und 36 ml 20%iger wäßriger Salzsäure für 6 Stunden und erhält die entsprechende 4-[4-(1,2,4-Triazolyl)]benzoesäure

Die nachfolgende Umsetzung einer 4-[4-(1,2,4-Triazolyl)]benzoesäure mit Guanidin nach vorhergehender Aktivierung mit Carbonyldiimidazol liefert gemäß der Allgemeinen Vorschrift A zur Herstellung von Benzoylguanidinen die entsprechenden 4-[4-(1,2,4-Triazolyl)]benzoyl-guanidine bzw. deren Salze:

### Beispiel 14: 3,5-Dibrom-4-[2-(1,2,4-triazolyl)]benzoyl-guanidin-dihydrochlorid

erhält man gemäß Allgemeiner Vorschrift A aus 3,5-Dibrom-4-[4-(1,2,4-triazolyl)]benzoesäure, farblose Kristalle, Schmp. > 290°C.

Die 3,5-Dibrom-4-[4-(1,2,4-triazolyl)benzoesäure (Fp. 281 - 282°C) erhält aus 3,5-Dibrom-4-[4-(1,2,4-triazolyl)]benzoesäure-methylester (Fp: 202 - 204°C) analog der allgemeinen Vorschrift zur Herstellung von 4-[4-(1,2,4-triazolyl)]benzoesäuren.

### Beispiel 15: 4-(1-Imidazolyl)-3-trifluormethyl-benzoylguanidin-dihydrochlorid: Farblose Kristalle, Smp. 244 - 48°C Zers.

### Syntheseweg:

a) 4-Fluor-3-trifluormethyl-benzoylguanidin aus 4-Fluor-3-trifluormethyl-benzoesäure und Guanidin nach Variante A, farbloses Pulver, Smp. 136 - 38°C.
b) 4-Imidazolyl-3-trifluormethyl-benzoylguanidin-dihydrochlorid aus a) durch Erhitzen (120°C) in DMF in Gegenwart von 2 eq Imidazol für 7 h, Abdestillieren des Lösemittels und Verreiben des Rückstands mit Wasser. Salzbildung analog Variante A.

## Patentansprüche

1. Benzoylguanidin der Formel I 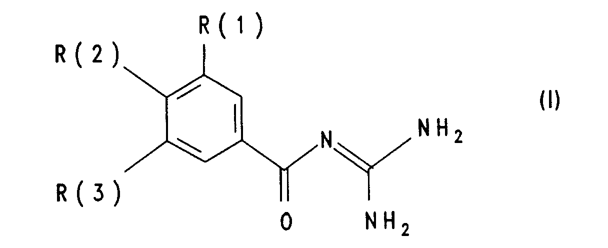 worin bedeuten:
R(1) Wasserstoff, F, Cl, Br, I, -NO₂, -C ≡ N, R(16)-CₚH₂ₚ-O_{q}, R(4)-SOₘ oder R(5)R(6)N-SO₂-;
m Null, 1 oder 2;
p Null oder 1;
q Null oder 1;
R(16) CᵣF₂ᵣ₊₁;
r 1, 2 oder 3;
R(4) und R(5)
(C₁-C₈)-Alkyl, (C₃-C₆)-Alkenyl, -CₙH₂ₙ-R(7) oder CF₃;
n Null, 1, 2, 3 oder 4;
R(7) (C₃-C₇)-Cycloalkyl oder Phenyl,
welches nicht substituiert ist oder substituiert mit 1 - 3 Substituenten aus der Gruppe F, Cl, CF₃, Methyl, Methoxy oder NR(8)R(9);
R(8) und R(9)
H oder C₁-C₄-Alkyl;
oder
R(5) auch H;
R(6) H oder (C₁-C₄)-Alkyl;
oder
R(5) und R(6)
gemeinsam 4 oder 5 Methylengruppen, von denen eine CH₂-Gruppe durch Sauerstoff, S, NH, N-CH₃ oder N-Benzyl ersetzt sein kann;
R(2) (C₁-C₉)-Stickstoff-Heteroaryl,
das über C oder N verknüpft ist und das unsubstituiert oder substituiert ist mit 1 - 3 Substituenten aus der Gruppe F, Cl, CF₃, CH₃, Methoxy, Hydroxy, Amino, Methylamino oder Dimethylamino;
oder
R(2) -SR(10), -OR(10) oder -NR(10)R(11);
R(10) -CₐH₂ₐ-(C₁-C₉)-Stickstoff-Heteroaryl,
das unsubstituiert oder substituiert ist mit 1 - 3 Substituenten aus der Gruppe F, Cl, CF₃, CH₃, Methoxy, Hydroxy, Amino, Methylamino, Dimethylamino;
a Null, 1 oder 2;
R(11) und R(12)
unabhängig voneinander wie R(10) definiert oder Wasserstoff oder (C₁-C₄)-Alkyl;
R(3) wie R(1) definiert, oder (C₁-C₆)-Alkyl oder -X-R(13);
X Sauerstoff, S oder NR(14);
R(14)
H oder (C₁-C₃)-Alkyl;
R(13)
H, (C₁-C₆)-Alkyl, (C₃-C₈)-Cycloalkyl oder -C_{b}H_{2b}-R(15);
b Null, 1, 2, 3 oder 4;
oder
R(13) und R(14)
gemeinsam 4 oder 5 Methylengruppen, von denen eine CH₂-Gruppe durch Sauerstoff, S, NH, N-CH₃ oder N-Benzyl ersetzt sein kann;
R(15) Phenyl,
das unsubstituiert oder substituiert ist mit 1 - 3 Substituenten aus der Gruppe F, Cl, CF₃, Methyl, Methoxy oder NR(8)R(9);
R(8) und R(9)
H oder (C₁-C₄)-Alkyl; sowie deren pharmazeutisch verträgliche Salze; mit Ausnahme von Verbindungen I, in denen gleichzeitig bedeuten:
R(1) R(4)-SOₘ oder R(5)R(6)NSO₂, und
R(2) (C₁-C₉)-Heteroaryl,
sowie mit Ausnahme der Verbindung 4-(3-Pyridyloxy)-3-N-pyrrolidinobenzoesäure-guanidin;

2. Verbindung der Formel I nach Anspruch 1, in der bedeuten:
R(1) Wasserstoff, F, Cl, -C ≡ N, R(16)-CₚH₂ₚ-O_{q}, R(4)-SOₘ oder R(5)R(6)N-SO₂-;
p Null oder 1;
q Null oder 1;
R(16) CᵣF₂ᵣ₊₁;
r 1, 2 oder 3;
m Null, 1 oder 2;
R(4) und R(5)
(C₁-C₈)-Alkyl, (C₃-C₄)-Alkenyl, -CₙH₂ₙ-R(7) oder -CF₃;
n Null oder 1;
R(7) (C₃-C₆)-Cycloalkyl oder Phenyl,
welches unsubstituiert ist oder substituiert mit 1-3 Substituenten aus der Gruppe F, Cl, CF₃, Methyl, Methoxy oder NR(8)R(9);
R(8) und R(9)
H oder Methyl;
oder
R(5) H;
R(6) H oder Methyl;
R(3) Wasserstoff, Methyl, Cyano, -CF₃, F oder Cl;
und die übrigen Reste wie in Anspruch 1 definiert sind.

3. Verbindung der Formel I nach Anspruch 1, in der bedeuten:
R(1) Wasserstoff, F, Cl, -C ≡ N, -CF₃, R(4)-SOₘ oder R(5)R(6)N-SO₂-;
m Null, 1 oder 2;
R(4) Methyl oder CF₃;
oder
R(5) und R(6)
unabhängig voneinander H oder Methyl;
R(2) (C₁-C₉)-Stickstoff-Heteroaryl,
das über C oder N verknüpft ist und das unsubstituiert oder substituiert ist mit einem Rest aus der Reihe F, Cl, CF₃, CH₃, Methoxy und Dimethylamino;
oder
R(2) -SR(10), -OR(10) oder -NR(10)R(11);
R(10) -CₐH₂ₐ-(C₁-C₉)-Stickstoff-Heteroaryl, das unsubstituiert oder substituiert ist mit einem Rest aus der Reihe F, Cl, CF₃, CH₃, Methoxy und Dimethylamino;
a Null, 1 oder 2;
R(11) und R(12)
unabhängig voneinander Wasserstoff oder Methyl;
R(3) Methyl, Cyano, Trifluormethyl, F, Cl oder Wasserstoff.

4. Verfahren zur Herstellung einer Verbindung I nach Anspruch 1, dadurch gekennzeichnet, daß man
eine Verbindung der Formel II 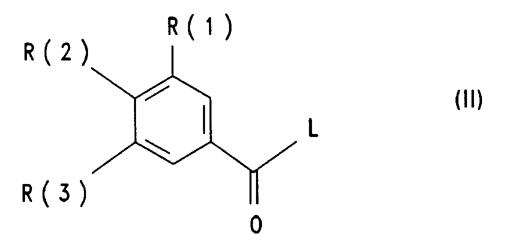 mit Guanidin umsetzt, worin R(1) bis R(3) die in Anspruch 1 angegebene Bedeutung besitzen und L für eine leicht nucleophil substituierbare leaving group steht.

5. Verwendung einer Verbindung 1 nach Anspruch 1 zum Herstellen eines Medikaments zum Behandeln von Herzrhythmusstörungen.

6. Verwendung einer Verbindung 1 nach Anspruch 1 zum Herstellen eines cardioprotektiven Medikaments zur Infarktprophylaxe und der Behandlung des Infarkts und der angina pectoris.

7. Medikament zum Behandeln von Herzrhythmusstörungen, des Infarkts und zur Cardioprotektion, dadurch gekennzeichnet, daß es eine wirksame Menge einer Verbindung I nach Anspruch 1 enthält.

## Claims

1. A benzoylguanidine of the formula I 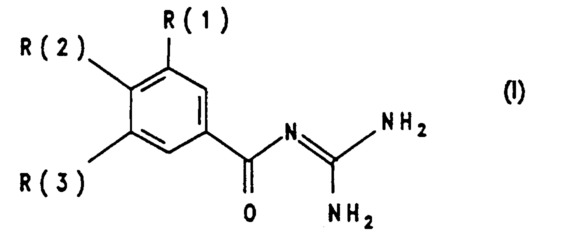 in which
R(1) is hydrogen, F, Cl, Br, I, -NO₂, -C≡N, R(16)-CₚH₂ₚ-O_{q}, R(4)-SOₘ or R(5)R(6)N-SO₂-;
m is zero, 1 or 2;
p is zero or 1;
q is zero or 1;
R(16) is CᵣF₂ᵣ₊₁;
r is 1, 2 or 3;
R(4) and R(5) are (C₁-C₈)-alkyl, (C₃-C₆)-alkenyl, -CₙH₂ₙ-R(7) or CF₃;
n is zero, 1, 2, 3 or 4;
R(7) is (C₃-C₇)-cycloalkyl or phenyl which is unsubstituted or substituted by 1-3 substituents selected from the group comprising F, Cl, CF₃, methyl, methoxy or NR(8)R(9);
R(8) and R(9) are H or C₁-C₄-alkyl; or R(5) is also H;
R(6) is H or (C₁-C₄)-alkyl; or
R(5) and R(6) together are 4 or 5 methylene groups, of which one CH₂ group can be replaced by oxygen, S, NH, N-CH₃ or N-benzyl;
R(2) is (C₁-C₉)-nitro-heteroaryl which is linked via C or N and which is unsubstituted or substituted by 1-3 substituents selected from the group comprising F, Cl, CF₃, CH₃, methoxy, hydroxyl, amino, methylamino or dimethylamino; or
R(2) is -SR(10), -OR(10) or -NR(10)R(11);
R(10) is -CₐR₂ₐ-(C₁-C₉)-nitro-heteroaryl which is unsubstituted or substituted by 1-3 substituents selected from the group comprising F, Cl, CF₃, CH₃, methoxy, hydroxyl, amino, methylamino, dimethylamino,
a is zero, 1 or 2;
R(11) and R(12) independently of one another have the definition given for R(10) or are hydrogen or (C₁-C₄)-alkyl;
R(3) has the definition given for R(1) or is (C₁-C₆)-alkyl or -X-R(13);
X is oxygen, S or NR(14);
R(14) is H or (C₁-C₃)-alkyl;
R(13) is H, (C₁-C₆)-alkyl, (C₃-C₈)-cycloalkyl or -C_{b}H_{2b}-R(15);
b is zero, 1, 2, 3 or 4; or
R(13) and R(14) together are 4 or 5 methylene groups, of which one CH₂ group can be replaced by oxygen, S, NH, N-CH₃ or N-benzyl;
R(15) is phenyl which is unsubstituted or substituted by 1-3 substituents selected from the group comprising F, Cl, CF₃, methyl, methoxy or NR(8)R(9);
R(8) and R(9) are H or (C₁-C₄)-alkyl;
and the pharmaceutically acceptable salts thereof; with the exception of compounds I in which, simultaneously,
R(1) is R(4)-SOₘ or R(5)R(6)NSO₂, and
R(2) is (C₁-C₉)-heteroaryl,
and also with the exception of the compound 4-(3-pyridyloxy)-3-N-pyrrolidinobenzoyl guanidine.

2. A compound of the formula I as claimed in claim 1, in which
R(1) is hydrogen, F, Cl, -C≡N, R(16)-CₚH₂ₚ-O_{q}, R(4)-SOₘ or R(5)R(6)N-SO₂-;
p is zero or 1;
q is zero or 1;
R(16) is CᵣF₂ᵣ₊₁;
r is 1, 2 or 3;
m is zero, 1 or 2;
R(4) and R(5) are (C₁-C₈)-alkyl, (C₃-C₄)-alkenyl, -CₙH₂ₙ-R(7) or -CF₃,
n is zero or 1,
R(7) is (C₃-C₆)-cycloalkyl or phenyl which is unsubstituted or substituted by 1-3 substituents selected from the group comprising F, Cl, CF₃, methyl, methoxy or NR(8)R(9);
R(8) and R(9) are H or methyl; or
R(5) is H;
R(6) is H or methyl;
R(3) is hydrogen, methyl, cyano, -CF₃, F or Cl; and the remaining radicals are as defined in claim 1.

3. A compound of the formula I as claimed in claim 1, in which
R(1) is hydrogen, F, Cl, -C≡N, -CF₃, R(4)-SOₘ or R(5)R(6)N-SO₂-;
m is zero, 1 or 2;
R(4) is methyl or CF₃; or
R(5) and R(6) independently of one another are H or methyl;
R(2) is (C₁-C₉)-nitro-heteroaryl which is linked via C or N and which is unsubstituted or substituted by a radical selected from the series comprising F, Cl, CF₃, CH₃, methoxy and dimethylamino; or
R(2) is -SR(10), -OR(10) or -NR(10)R(11);
R(10) is -CₐH₂ₐ-(C₁-C₉)-nitro-heteroaryl which is unsubstituted or substituted by a radical selected from the series comprising F, Cl, CF₃, CH₃, methoxy and dimethylamino;
a is zero, 1 or 2;
R(11) and R(12) independently of one another are hydrogen or methyl;
R(3) is methyl, cyano, trifluoromethyl, F, Cl or hydrogen.

4. A process for the preparation of a compound I as claimed in claim 1, which comprises reacting a compound of the formula II 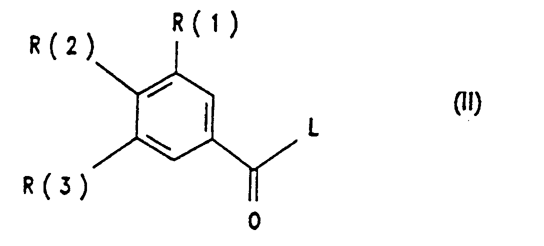 in which R(1) to R(3) are as defined in claim 1 and L is a leaving group which can readily be substituted by a nucleophile, with guanidine.

5. The use of a compound I as claimed in claim 1 for the preparation of a pharmaceutical for the treatment of cardiac arrhythmias.

6. The use of a compound I as claimed in claim 1 for the preparation of a cardioprotective pharmaceutical for the prophylaxis of infarctions, and for the treatment of infarctions and of angina pectoris.

7. A pharmaceutical for the treatment of cardiac arrhythmias, of infarction and for cardioprotection, which comprises an effective amount of a compound I as claimed in claim 1.

## Revendications

1. Benzoylguanidine de formule I 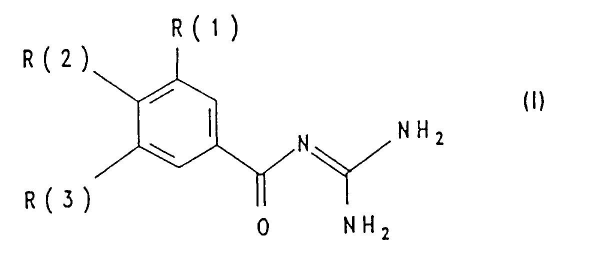 dans laquelle
R(1) représente un atome d'hydrogène ou de F, Cl, Br, I, ou un groupe -NO₂, -C≡N, R(16)-CₚH₂ₚ-O_{q}, R(4)-SOₘ ou R(5)R(6)N-SO₂-,
m est zéro, 1 ou 2,
p est zéro ou 1,
q est zéro ou 1,
R(16) représente CᵣF₂ᵣ₊₁,
r est 1, 2 ou 3,
R(4) et R(5) représentent
un groupe alkyle en C₁-C₈, alcényle en C₃-C₆, -CₙH₂ₙ-R(7) ou CF₃,
n est zéro, 1, 2, 3 ou 4,
R(7) représente un groupe cycloalkyle en C₃-C₇, ou phényle
qui est non substitué ou substitué par 1-3 substituants choisis parmi F, Cl, CF₃, le groupe méthyle, méthoxy ou NR(8)R(9),
R(8) et R(9) représentant
H ou un groupe alkyle en C₁-C₄,
ou
R(5) représente également H,
R(6) représente H ou un groupe alkyle en C₁-C₄,
ou
R(5) et R(6) représentent ensemble 4 ou 5 groupes méthylène, dont un groupe CH₂ peut être remplacé par un atome d'oxygène ou de S ou par un groupe NH, N-CH₃ ou N-benzyle,
R(2) représente un groupe N-hétéroaryle en C₁-C₉,
qui est lié par C ou N est qui est non substitué ou substitué par 1-3 substituants choisis parmi les substituants F, Cl, CF₃, CH₃, méthoxy, hydroxy, amino, méthylamino ou diméthylamino,
ou
R(2) représente -SR(10), -OR(10), ou -NR(10)R(11),
R(10) représente un groupe -CₐH₂ₐ-N-hétéroaryle(C₁-C₉) qui est non substitué ou substitué par 1-3 substituants choisis parmi les substituants F, Cl, CF₃, CH₃, méthoxy, hydroxy, amino, méthylamino, diméthylamino,
a est zéro, 1 ou 2,
R(11) et R(12)
indépendamment l'un de l'autre, sont définis comme R(10) ou représentent un atome d'hydrogène ou un groupe alkyle en C₁-C₄,
R(3) est défini comme R(1), ou représente un groupe alkyle en C₁-C₆ ou -X-R(13),
X représente un atome d'oxygène, de S ou NR(14),
R(14) représente
H ou un groupe alkyle en C₁-C₃,
R(13) représente
H ou un groupe alkyle en C₁-C₆, cycloalkyle en
C₃-C₈ ou -C_{b}H_{2b}-R(15),
b est zéro, 1, 2, 3 ou 4,
ou
R(13) et R(14)
représentent ensemble 4 ou 5 groupes méthylène, dont un groupe CH₂ peut être remplacé par un atome d'oxygène ou de S ou par un groupe NH,
N-CH₃ ou N-benzyle,
R(15) représente un groupe phényle qui est non substitué ou substitué par 1-3 substituants choisis parmi F, Cl, CF₃, méthyle, méthoxy ou NR(8)R(9),
R(8) et R(9)
représentant H ou un groupe alkyle en C₁-C₄,
et ses sels pharmaceutiquement acceptables, à l'exclusion des composés I dans lesquels simultanément
R(1) représente R(4)-SOₘ ou R(5)R(6)NSO₂, et
R(2) représente un groupe hétéroaryle en C₁-C₉,
ainsi qu'à l'exclusion du composé 4-(3-pyridyloxy)-3-N-pyrrolidinobenzoylguanidine.

2. Composé de formule I selon la revendication 1, dans lequel
R(1) représente un atome d'hydrogène ou de F, Cl, -C≡N, R(16)-CₚH₂ₚ-O_{q}, R(4)-SOₘ ou R(5)R(6)N-SO₂-,
p est zéro ou 1,
q est zéro ou 1,
R(16) représente CᵣF₂ᵣ₊₁,
r est 1, 2 ou 3,
m est zéro, 1 ou 2,
R(4) et R(5) représentent
un groupe alkyle en C₁-C₈, alcényle en C₃-C₄, -CₙH₂ₙ-R(7) ou -CF₃,
n est zéro ou 1,
R(7) représente un groupe cycloalkyle en C₃-C₆ ou phényle
qui est non substitué ou substitué par 1-3 substituants choisis parmi F, Cl, CF₃, le groupe méthyle, méthoxy ou NR(8)R(9),
R(8) et R(9) représentant
H ou le groupe méthyle,
ou
R(5) est H,
R(6) est H ou le groupe méthyle,
R(3) représente un atome d'hydrogène ou de F, Cl ou le groupe méthyle, cyano ou -CF₃,
et les autres radicaux sont tels que définis dans la revendication 1.

3. Composé de formule I selon la revendication 1 dans lequel
R(1) représente un atome d'hydrogène ou de F, Cl, -C≡N, -CF₃, R(4)-SOₘ ou R(5)R(6)N-SO₂-,
m est zéro, 1, 2,
R(4) représente le groupe méthyle ou CF₃,
R(5) et R(6) représentent, indépendamment l'un de l'autre,
H ou le groupe méthyle;
R(2) représente un groupe N-hétéroaryle en C₁-C₉,
qui est lié par C ou N est qui est non substitué ou substitué par un substituant choisi parmi les substituants F, Cl, CF₃, CH₃, méthoxy et diméthylamino,
ou
R(2) représente -SR(10), -OR(10), ou -NR(10)R(11),
R(10) représente un groupe -CₐH₂ₐ-N-hétéroaryle(C₁-C₉) qui est non substitué ou substitué par un substituant choisi parmi les substituants F, Cl, CF₃, CH₃, méthoxy et diméthylamino,
a est zéro, 1 ou 2,
R(11) et R(12)
représentent, indépendamment l'un de l'autre, un atome d'hydrogène ou le groupe méthyle,
R(3) représente un atome d'hydrogène ou de F, Cl ou le groupe méthyle, cyano ou trifluorométhyle.

4. Procédé pour la préparation d'un composé I selon la revendication 1, caractérisé en ce que l'on fait réagir avec de la guanidine un composé de formule II 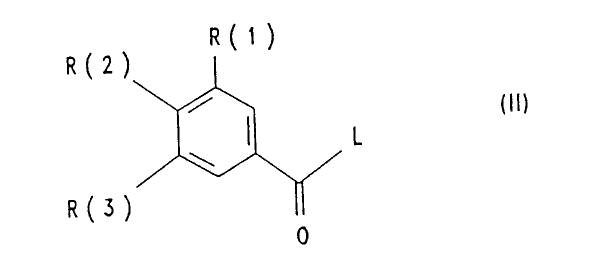 dans laquelle R(1) à R(3) ont les significations données dans la revendication 1, et L représente un groupe partant aisément remplaçable par substitution nucléophile.

5. Utilisation d'un composé I selon la revendication 1, pour la fabrication d'un médicament destiné au traitement de troubles du rythme cardiaque.

6. Utilisation d'un composé I selon la revendication 1, pour la fabrication d'un médicament cardioprotecteur destiné à la prophylaxie de l'infarctus et au traitement de l'infarctus et de l'angine de poitrine.

7. Médicament destiné au traitement de troubles du rythme cardiaque, de l'infarctus et à la protection cardiaque, caractérisé en ce qu'il contient une quantité efficace d'un composé I selon la revendication 1.
